# EUROPEAN PATENT APPLICATION

(11) **EP 3 649 977 A1**
(43) Date of publication of application: **13.05.2020**
(21) Application number: 19207767.5
(22) Date of filing: 07.11.2019
(51) Int. Cl.: A61B 90/00, A61B 90/96, A61F 2/32, G06K 19/06, A61F 2/30

(54) **MEDICAL IMPLANT WITH 3D PRINTED CODE FEATURE**

(30) Priority: 07.11.2018 US 201816182831
(71) Applicant: Ethicon LLC, 00969 Guaynabo (PR)
(72) Inventor: KAVANAGH, Edward P., Ringaskiddy, P43ED82 (IE); DEVLIN, Aoife M., Ringaskiddy, P43ED82 (IE)
(74) Representative: Carpmaels & Ransford LLP

(57) **Abstract**

A medical implant includes a body and an identification assembly. The body includes a first material that is biocompatible, an inner portion, and an external surface made form the first material. The external surface completely contains the interior portion of the body such that the external surface is the only portion of the body exposed to an external environment. The identification assembly is fixed relative to the body. The identification assembly includes a radiopaque material forming a datamatrix. The identification assembly stores a readable data associated with the medical implant. The identification assembly may transfer the readable data to a scanning system.

## Description

### BACKGROUND

Medical implants are devices or tissues that are placed inside or on the surface of the body. Many implants are prosthetics, which are intended to replace missing body parts. Other implants deliver medication, monitor body functions, or provide support to organs and tissues.

Three-dimensional (3D) printing is an additive printing process used to make three-dimensional solid objects from a digital model. 3D printing may be used in various processes including but not limited to rapid product prototyping, product manufacturing, mold generation, and mold master generation. 3D printing techniques are considered additive processes because they involve the application of successive layers of material. This is unlike traditional machining processes, which often rely upon the removal of material to create the final object. Various materials may be used in 3D printing. For example, materials such as polymide, alumide, titanium, or thermoplastic polyurethane may be used in 3D printing. Some 3D printing techniques utilize powder as the basic material, then transform the powder into a desired shape or structures to form a product. For example, laser sintering involves applying successive thin layers of powder, one layer on top of the next. Between application of each layer of powder, a laser travels over desired portions of the current powder layer and sinters targeted powder together, eventually forming the desired shape or structure. Once complete, the final product may be removed from the unsintered powder.

While various kinds of medical implants have been made and used, it is believed that no one prior to the inventor(s) has made or used the invention described in the appended claims.

### BRIEF DESCRIPTION OF THE DRAWINGS

While the specification concludes with claims which particularly point out and distinctly claim this technology, it is believed this technology will be better understood from the following description of certain examples taken in conjunction with the accompanying drawings, in which like reference numerals identify the same elements and in which:
FIG. 1 depicts a perspective view of an exemplary hip replacement prosthetic;
FIG. 2 depicts an exploded perspective view of the exemplary hip replacement prosthetic of FIG. 1;
FIG. 3 depicts a front elevational view of an exemplary medical implant having an embedded identification assembly;
FIG. 4 depicts a cross-sectional view of the medical implant of FIG. 3, taken along line 4-4 of FIG. 3;
FIG 5 depicts a cross-sectional view of the medical implant of FIG. 3, taken along line 5-5 of FIG. 3;
FIG. 6 depicts a top plan view of the embedded identification assembly of FIG. 3;
FIG. 7A depicts a side cross-sectional view of a 3D printer manufacturing a portion of the medical implant of FIG. 3;
FIG. 7B depicts a side cross-sectional view of the 3D printer of FIG. 7A manufacturing another portion of the medical implant of FIG. 3;
FIG. 7C depicts a side cross-sectional view of the 3D printer of FIG. 7A manufacturing a portion of the embedded identification assembly of the medical implant of FIG. 3;
FIG. 7D depicts a side cross-sectional view of the 3D printer of FIG. 7A manufacturing another portion of the embedded identification assembly of the medical implant of FIG. 3;
FIG. 7E depicts a side cross-sectional view of the 3D printer of FIG. 7A manufacturing another portion of the embedded identification assembly of the medical implant of FIG. 3;
FIG. 7F depicts a side cross-sectional view of the 3D printer of FIG. 7A manufacturing another portion of the embedded identification assembly of the medical implant of FIG. 3;
FIG. 7G depicts a side cross-sectional view of the 3D printer of FIG. 7A manufacturing another portion of the embedded identification assembly of the medical implant of FIG. 3; and
FIG. 7H depicts a side cross-sectional view of the 3D printer of FIG. 7A and a completely manufactured medical implant of FIG. 3.

The drawings are not intended to be limiting in any way, and it is contemplated that various embodiments of the technology may be carried out in a variety of other ways, including those not necessarily depicted in the drawings. The accompanying drawings incorporated in and forming a part of the specification illustrate several aspects of the present technology, and together with the description serve to explain the principles of the technology; it being understood, however, that this technology is not limited to the precise arrangements shown.

### DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

### I. Exemplary Prosthetic Medical Implant

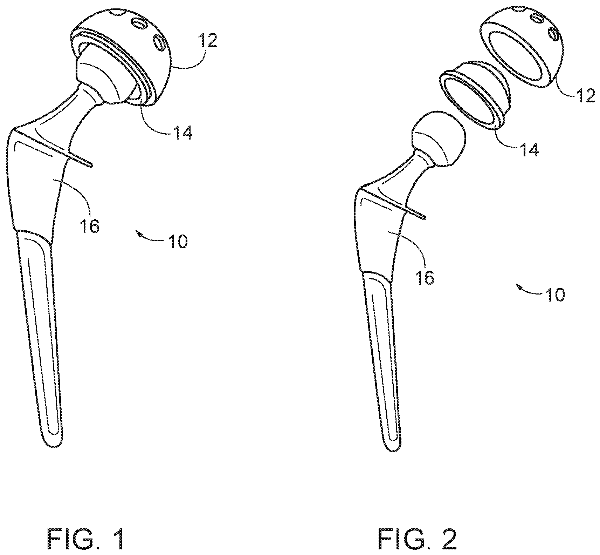

As mentioned above, prosthetic implants may be implanted inside or on the surface of the body with the intention of replacing missing body parts or for other purposes. Some non-limiting examples of prosthetic implants include bone screws, bone plates, total knee replacement prosthetics, unicompartmental knee arthroplasty prosthetics, hip replacement prosthetics, etc. FIGS. 1-2 show an exemplary hip replacement prosthetic (10). Hip replacement prosthetic (10) may be suitably implanted into a patient to replace a damaged hip. Hip replacement prosthetic (10) includes an acetabular component (12), a plastic liner (14), and a femoral component (16).

Acetabular component (12) of hip replacement prosthetic (10) may be suitably affixed to a pelvis while femoral component (16) may be suitably affixed to a femur. Plastic liner (14) may serve as an intermediary between a top end of femoral component (16) and the ball socket cavity of acetabular component (12). When suitably implanted, femoral component (16) may move relative to acetabular component (12). Additionally, when suitably implanted, femoral component (16) and acetabular component (12) may transfer loads between each other.

It should be understood that, like many prosthetic implants, hip replacement prosthetic (10) is intended to be permanently implanted within the patient. Therefore, once hip replacement prosthetic (10) is implanted within the body of a patient, re-accessing different elements of prosthetic (10) may require a new surgical procedure.

### II. Alternative Medical Implant with 3D Printed Identification Feature

In some instances, it may be desirable to track a prosthetic medical implant. After a prosthetic medical implant has been implanted within a patient for a prolonged period of time, a physician may wish to easily obtain information associated with a specific medical implant for various reasons as would be apparent to one having ordinary skill in the art in view of the teachings herein. For instance, it may be desirable to identify certain characteristics of a prosthetic medical implant already suitably implanted within a patient. One exemplary reason may be to identify what exact materials were used in manufacturing the prosthetic medical implant. However, as mentioned above, once a medical prosthetic implant is suitably implanted, re-accessing a prosthetic implant, even for identification purposes, may require a new surgical procedure. Therefore, it may be desirable to have a medical implant that may be identifiable utilizing non-invasive means.

Various kinds of prosthetic implants may be manufactured using 3D printing technologies. In addition to manufacturing various kinds of prosthetic implants, 3D printing technologies may incorporate various materials while manufacturing a single prosthetic implant. Therefore, in instances where 3D printing includes applying successive layers of manufacturing powder, then sintering desired portions of powder between application of successive layers, various types of manufacturing powder may be incorporated into a single layer of manufacturing powder. For example, a first powder having a first range of material characteristics and a second powder having a second range of material characteristics, which are different than the first powder, may both be applied on a single layer and selectively sintered to form a desired object/shape. Suitable methods and means of applying layers of powder having multiple powder materials will be apparent to one having ordinary skill in the art in view of the teachings herein.

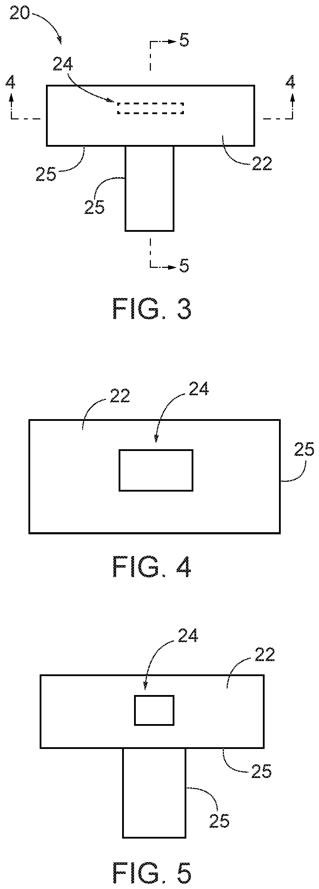

FIGS. 3-5 show an exemplary prosthetic medical implant (20) that may be used for any suitable purpose as would be apparent to one having ordinary skill in the art in view of the teachings herein. For example, medical implant (20) may be dimensioned and configured for use in an exemplary hip replacement prosthetic (10), a total knee replacement prosthetic, etc. Regardless of function, prosthetic medical implant (20) may be dimensioned and configured to be implanted within a patient, either permanently or for a prolonged period of time.

Medical implant (20) may be manufactured using 3D printed technologies. Medical implant (20) includes a body (22) defining an external perimeter (25), and an identification assembly (24). At least the external perimeter (25) of body (22) is made from a biocompatible material. Any suitable biocompatible material may be used as would be apparent to one having ordinary skill in the art in view of the teachings herein. Additionally, other suitable portions of body (22) and/or identification assembly (24) may be made of biocompatible material as well.

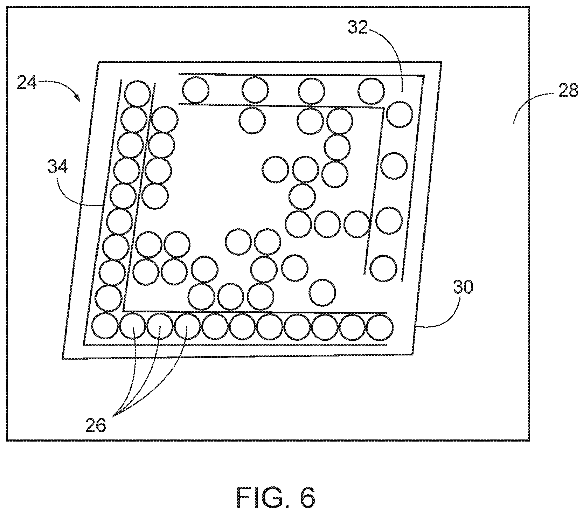

As will be described in greater detail below, identification assembly (24) contains readable information that may be obtained through non-invasively scanning identification assembly (24). As best seen in FIG. 6, the current example of identification assembly (24) includes a plurality of individual markers (26) arranged in a datamatrix on top of a base (28) to form a two-dimensional (2D) barcode. While in the current example, 2D barcode is a non-connected datamatrix, 2D barcode may also be a connected data matrix.

Individual markers (26) may define any suitable shape in contrast to base (28) as would be apparent to one having ordinary skill in the art in view of the teachings herein. For instance, in contrast to base (28), markers (26) may form bumps, recesses, dots, circles, squares, pyramids, etc. Markers (26) may be substantially flush with base (28) but made from a different material to contrast with base (28). Markers (26) may have any suitable dimension that would be apparent to one having ordinary skill in the art in view of the teachings herein. For instance, markers (26) may have a width and/or height of approximately 0.2 mm, 0.4 mm, 0.6 mm, 0.8 mm, 1 mm, 1.2 mm, etc. Likewise, individual markers (26) may be formed out of any suitable material in accordance with the description herein. Base (28) may be formed of a contrasting material compared to individual markers (26) such that markers (26) may be easily read (optically or otherwise), and such that spaces in datamatrix defined by base (28) may also be read. For example, base (28) may be made from the same material used to make body (22). In some instances, identification assembly (24) may contain readable information formed in 2D barcode made from one material, where individual markers (26) are recessed areas, rather than areas of additional material used to form datamatrix.

A portion of base (28) extending around the exterior perimeter of individual markers (26) forming datamatrix defines a suitable quiet zone (30) that is configured to ensure a suitable barcode reader does not pick up any information that is not pertinent to the 2D barcode formed by identification assembly (24). Quiet zone (30) thus serves as a blank margin surrounding the exterior perimeter of individual markers (26) forming datamatrix. Quiet zone (30) may have any suitable dimension as would be apparent to one having ordinary skill in the art in view of the teachings herein. Additionally, portions of individual markers (26) defining the exterior perimeter form a suitable clocking pattern (32) and finder pattern (34). Finder pattern (34) consists of individual markers (26) forming two lines on the outer perimeter of the datamatrix code that define an "L" shape. Finder pattern (34) allows a barcode reader to orient the code so that the bar code reader understands where to begin reading the pattern formed by individual markers (26) in datamatrix. Clocking pattern (32) is used to inform a barcode reader about the number modules within the datamatrix.

The portion of identification assembly (24) formed by individual markers (26) may be radiopaque in nature. Therefore, when attempting to obtain information of identification assembly (24), the operator may take an X-ray image of the portion of patient containing implant at a suitable angle. The radiopaque nature of the individual markers (26) may allow visualization of datamatrix formed by identification assembly (24) on an X-ray image. The portion of the X-ray image containing identification assembly (24) may then be scanned via a suitable optical reader to obtain the readable information contained within identification assembly (24). Identification assembly (24) may be configured to be used in conjunction with any suitable optical scanning/reading system as would be apparent to one having ordinary skill in the art in view of the teachings herein. In the current example, individual markers (26) are formed of radiopaque material, while base (28) is made of a contrasting material. However, this is merely optional, as base (28) may be formed of a radiopaque material, while markers (26) may be formed of another material. Alternatively, base (28) may be formed of a material having a first density of radiopaque material while markers (26) may be formed of a material having a second density of radiopaque material such that the base (28) and markers (26) visually contrast with each other.

Alternatively, identification assembly (24) may be non-invasively scannable via any suitable means as would be apparent to one having ordinary skill in the art in view of the teachings herein. For instance, identification assembly (24) may be suitably scanned using any other technique besides the use of an optical scanner. As just one non-limiting example, identification assembly (24) may be 3D printed to incorporate Radio Frequency Identification (RFID) circuits. Therefore, after implant (20) has been suitably implanted, the operator may wave an RFID wand over implant (20) or otherwise bring an RFID scanner into sufficient proximity with implant (20) and identification assembly (24) to read information contained within identification assembly (24).

One non-limiting example of RFID circuits that may be used in identification assembly (24) includes the use of planar circuit clipless RFID tags, which may be designed using standard planar microstrip, co-planar waveguide, stripline resonant structures such as antennas, filters, and fractals, etc. Planar circuit chipless RFID tags may include a number of dipole antennas that resonate at different frequencies. When identification assemblies (24) made from planar circuit chipless RFIS tags are exposed to a frequency sweep signal, the reader looks for magnitude dips in the spectrum as a result of the dipoles. Each dipole may have a 1:1 correspondence to a data bit.

In addition to or in the alternative of RFID circuits being used, microwave readable dielectric barcodes may be incorporated into identification assembly (24). In such instances, a unique dielectric barcode, or other suitable unique pattern, may be implemented into identification assembly (24), which may be different for every medical implant (20). The microwave readable dielectric barcode may be formed from dielectric ink, dielectric powder, or any other suitable microwave readable dielectric material that would be apparent to one having ordinary skill in the art in view of the teachings herein. The dielectric material that creates identification assembly (24) may be exposed to a high frequency microwave signals (e.g., >10 GHz), which is reflected by areas of identification assembly (24) composed of dielectric material, which can then be detected by a suitable reader. This may provide a reading range of identification assembly (24) from a substantial distance (e.g., up to 4 feet away).

Microwave readable dielectric barcodes or tags may be printed directly on a layer of medical implant (20) during the manufacturing process. Alternatively, microwave readable dielectric barcodes or tags could be pre-printed on some other sheet of material, that is later incorporated into medical implant (20) during the 3D printing process. In such instances the pre-printed sheet may be overlaid onto an already-3D-printed layer of medical implant (20), with additional layers then being 3D-printed on top of the pre-printed sheet. Of course, any other suitable method of forming identification assembly (24) with microwave readable dielectric barcodes/tags may be used as would be apparent to one having ordinary skill in the art in view of the teachings herein.

Microwave readable dielectric barcodes may be further constructed in accordance with at least some of the teachings of U.S. App. No. 2009/0039158, entitled "Microwave Readable Dielectric Barcode," abandoned, published on February 12, 2009, the disclosure of which is incorporated by reference herein; and/or in accordance with other configurations as would be apparent to one of ordinary skill in the art in view of the teachings herein.

As best seen in FIGS. 3-5 of the current example, identification assembly (24) is completely embedded within body (22) such that identification assembly (24) does not form any portion of external perimeter (25). Because identification assembly (24) of the current example is completely embedded within body (22), identification assembly (24) may contain materials that are not biocompatible, such as RFID circuits or radiopaque materials described above. Moreover, embedding identification assembly (24) within body (22) may prevent the presence of identification assembly (24) from having potentially undesirable effects on the surface characteristics of body (22), which may be particularly important to the success of an implant (20) depending on the kind and location of implant (20) in the patient's body. In some variations, identification assembly (24) may be made from biocompatible materials that are identical to or different than the materials used to form body (22). Also in some variations, identification assembly (24) is provided directly on an exterior surface of implant (20), such that identification assembly (24) need not necessarily be embedded within body (22) in all cases.

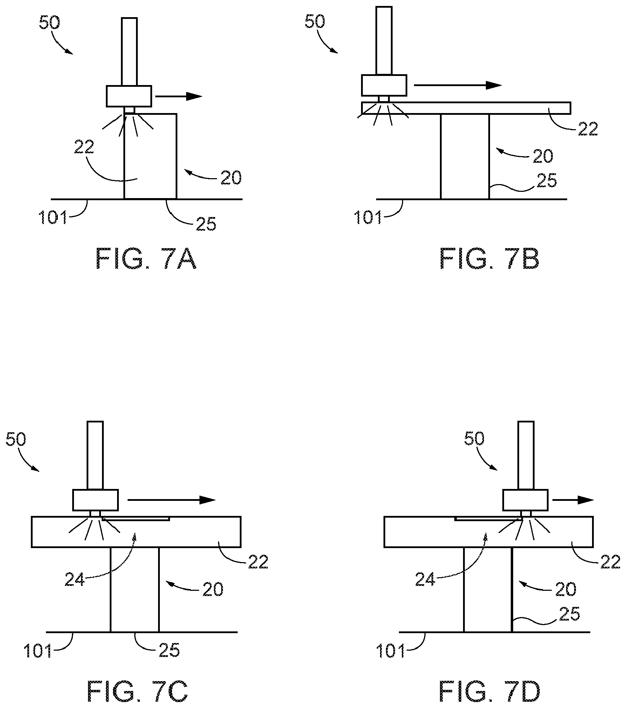

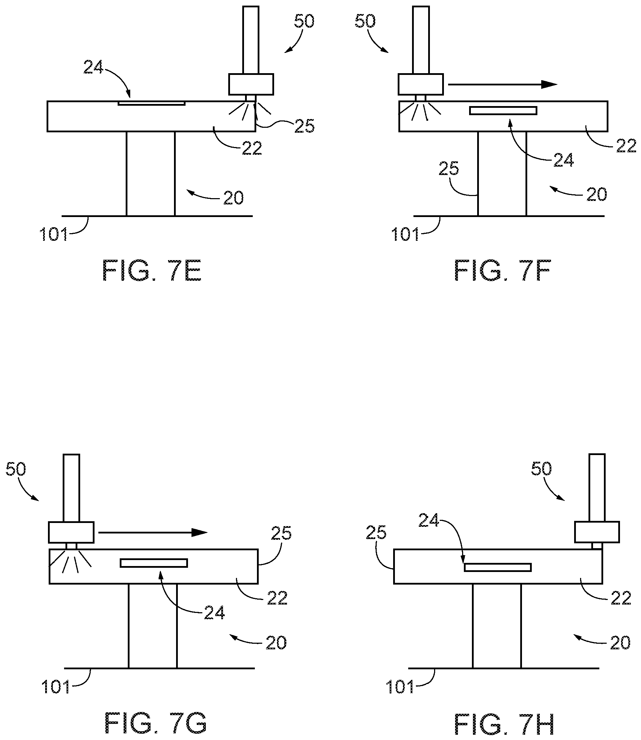

FIGS. 7A-7H show an exemplary method of manufacturing implant (20) having identification assembly (24) embedded within biocompatible body (22). First, as exemplified in FIG. 7A, 3D printer (50) adds a layer of powder on a base plate (101), then traverses across the recently added layer of powder while sintering desired portions of powder used to form body (22) of implant (20). 3D printer (50) may apply layers of powder using any suitable technique as would be apparent to one having ordinary skill in the art in view of the teachings herein. As shown in FIG. 7A, while 3D printer (50) is forming the bottom portion of body (22), 3D printer (50) is applying powder for body (22) and sintering that powder across the entire cross-sectional area of implant (20), thereby forming a first portion of body (22).

As shown between FIGS. 7A-7B, 3D printer (50) repeats this additive process of applying layers of powder and sintering powder to form body (22) until reaching the portion of implant (20) where body (22) fully embeds identification assembly (24). Then, as exemplified between FIGS. 7C-7E, 3D printer (50) may apply a first powder forming body (22) as well as a second powder forming selected portions of identification assembly (24). As mentioned above, various types of manufacturing powder may be incorporated on a single layer of manufacturing powder. Therefore, the first powder having characteristics required for body (22) and base (28) may be applied on the same layer along with a second powder having characteristics required for markers (26). The first powder and the second powder may be applied on the same layer in a pattern such that markers (26) form their desired shape of datamatrix once sintered.

After 3D printer (50) applies and sinters layers of multiple powders to form identification assembly (24) embedded in body (22) as exemplified between FIGS. 7C-7E, 3D printer (50) may then apply and sinter successive layers of powder forming body (22) over identification assembly (24) until implant (20) is completed as exemplified between FIGS. 7F-7H.

In examples where RFID components are printed, a non-conductive layer of material may be printed first, then conductive materials may be printed on top of the non-conductive layer. An air space may be formed on top of the conductive layer, which is then enclosed with other 3D printed layers of biocompatible materials.

While two different powders are being used in the current example, any suitable number of powders may be used as would be apparent to one having ordinary skill in the art in view of the teachings herein. For instance, three, four, five, six, etc. powders may be used on a single layer in order to suitably form the components of identification assembly (24). Multiple powders may be applied and sintered on a single layer using any suitable method and means as would be apparent to one having ordinary skill in the art in view of the teachings herein. Additionally, suitable components for RFID may be 3D printed in replacement of markers (26) as would be apparent to one having ordinary skill in the art in view of the teachings herein.

It should be understood that while in the current example, 3D printer (50) adds an entire layer of powder on top of base plate (101), then traverses above and sinters desired portions of layers, this is entirely optional. 3D printer (50) may add powder on top of base plate (101) and then sinter desired powder using any suitable technique as would be apparent to one having ordinary skill in the art in view of the teachings herein.

While in the current example, implant (20) is 3D printed utilizing metallic powder as the basic material and sintering to form the desired shape, this is just one of many materials and methods that may be used. For example, implant (20) may be 3D printed utilizing a suitable plastic material and corresponding 3D printing technique. Any other suitable basic material and 3D printing technique may be used as would be apparent to one having ordinary skill in the art in view of the teachings herein. Further, it is envisioned that more items than just a prosthetic implant (20) may be 3D printed with a corresponding identification assembly (24).

### III. Exemplary Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

A medical implant, comprising: (a) a body, wherein the body comprises:(i) a first material, wherein the first material is biocompatible, (ii) an interior portion, and (iii) an external surface, wherein the external surface is made from the first material, wherein the external surface completely contains the interior portion of the body such that the external surface is the only portion of the body exposed to an external environment; and (b) an identification assembly fixed relative to the body, wherein the identification assembly comprises a radiopaque material, wherein the radiopaque material forms a datamatrix, wherein the identification assembly is configured to store a readable data associated with the medical implant, where the identification assembly is configured to transfer the readable data to a scanning system.

### Example 2

The medical implant of Example 1, wherein the datamatrix forms a two-dimensional barcode assembly.

### Example 3

The medical implant of Example 2, wherein the datamatrix comprises a non-connected datamatrix.

### Example 4

Th medical implant of any one or more of Examples 2 through 3, wherein the two-dimensional barcode assembly comprises a base and a plurality of individual markers.

### Example 5

The medical implant of Example 4, wherein the two-dimensional barcode assembly comprises a finder pattern and a clocking pattern formed from the plurality of individual markers.

### Example 6

The medical implant of Example 5, wherein the two-dimensional barcode assembly comprises a base, wherein an outer perimeter defined by the clocking pattern and the finder pattern and the base define a quite zone.

### Example 7

The medical implant of any one or more of Examples 4 through 6, wherein the plurality of individual markers each define a recessed area.

### Example 8

The medical implant of any one or more of Examples 4 through 7, wherein each marker in the plurality of individual markers comprises a bump.

### Example 9

The medical implant of any one or more of Examples 4 through 8, wherein the plurality of individual markers are formed from a second material that is different than the first material.

### Example 10

The medical implant of Example 9, wherein the second material comprises the radiopaque material.

### Example 11

The medical implant of any one or more of Examples 1 through 10, wherein the identification assembly is housed within the interior portion of the body such that the identification assembly is completely embedded within the body.

### Example 12

The medical implant of any one or more of Examples 1 through 11, wherein the medical implant comprises a hip replacement prosthetic.

### Example 13

The medical implant of any one or more of Examples 1 through 12, wherein the identification assembly comprise a radio frequency identification circuit embedded within the body.

### Example 14

The medical implant of Example 13, wherein the radio frequency identification circuit is associated with the interior portion of the body.

### Example 15

A medical implant, comprising: (a) a biocompatible body made from a first material, wherein the biocompatible body comprises: (i) an exterior surface, and (ii) an interior portion completely housed within the exterior surface; and (b) an identification assembly comprising a plurality of markers forming a datamatrix, wherein the datamatrix is housed within the interior portion of the medical implant, wherein the identification assembly is fixed relative to the biocompatible body, wherein the identification assembly is configured to store a data set associated with the medical implant, wherein the identification assembly is configured to be scanned by a scanning device in order to transmit the data set to the scanning device.

### Example 16

The medical implant of Example 15, wherein the plurality of markers are at least partially formed of a second material.

### Example 17

The medical implant of Example 16, wherein the second material is a radiopaque material.

### Example 18

The medical implant of any one or more of Examples 15 through 17, wherein each marker in the plurality of markers comprise a square shape.

### Example 19

A method of three-dimensionally printing a medical implant comprising a biocompatible body and an identification assembly, wherein the identification assembly is configured to transmit a data set to a scanning device, the method comprising: (a) three-dimensionally printing a first portion of the biocompatible body; (b) three-dimensionally printing a second portion of the biocompatible body and the identification assembly on top of the first portion of the biocompatible body; and (c) three-dimensionally printing a third portion of the biocompatible body on top of the second portion of the biocompatible body and the identification assembly such that the biocompatible body completely surrounds the identification assembly.

### Example 20

The method of Example 19, wherein printing the identification assembly further comprises printing a two-dimensional bar code.

### IV. Miscellaneous

It should also be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometries, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A medical implant, comprising:
(a) a body, wherein the body comprises:
(i) a first material, wherein the first material is biocompatible,
(ii) an interior portion, and
(iii) an external surface, wherein the external surface is made from the first material, wherein the external surface completely contains the interior portion of the body such that the external surface is the only portion of the body exposed to an external environment; and
(b) an identification assembly fixed relative to the body, wherein the identification assembly comprises a radiopaque material, wherein the radiopaque material forms a datamatrix, wherein the identification assembly is configured to store a readable data associated with the medical implant, where the identification assembly is configured to transfer the readable data to a scanning system.

2. The medical implant of claim 1, wherein the datamatrix forms a two-dimensional barcode assembly.

3. The medical implant of claim 2, wherein the datamatrix comprises a non-connected datamatrix.

4. The medical implant of claim 2, wherein the two-dimensional barcode assembly comprises a base and a plurality of individual markers.

5. The medical implant of claim 4, wherein the two-dimensional barcode assembly comprises a finder pattern and a clocking pattern formed from the plurality of individual markers.

6. The medical implant of claim 5, wherein the two-dimensional barcode assembly comprises a base, wherein an outer perimeter defined by the clocking pattern and the finder pattern and the base define a quite zone.

7. The medical implant of claim 4, wherein the plurality of individual markers each define a recessed area.

8. The medical implant of claim 4, wherein each marker in the plurality of individual markers comprises a bump.

9. The medical implant of claim 4, wherein the plurality of individual markers are formed from a second material that is different than the first material.

10. The medical implant of claim 9, wherein the second material comprises the radiopaque material.

11. The medical implant of claim 1, wherein the identification assembly is housed within the interior portion of the body such that the identification assembly is completely embedded within the body.

12. The medical implant of claim 1, wherein the medical implant comprises a hip replacement prosthetic.

13. The medical implant of claim 1, wherein the identification assembly comprise a radio frequency identification circuit embedded within the body.

14. The medical implant of claim 13, wherein the radio frequency identification circuit is associated with the interior portion of the body.

15. A medical implant, comprising:
(a) a biocompatible body made from a first material, wherein the biocompatible body comprises:
(i) an exterior surface, and
(ii) an interior portion completely housed within the exterior surface; and
(b) an identification assembly comprising a plurality of markers forming a datamatrix, wherein the datamatrix is housed within the interior portion of the medical implant, wherein the identification assembly is fixed relative to the biocompatible body, wherein the identification assembly is configured to store a data set associated with the medical implant, wherein the identification assembly is configured to be scanned by a scanning device in order to transmit the data set to the scanning device.

16. The medical implant of claim 15, wherein the plurality of markers are at least partially formed of a second material.

17. The medical implant of claim 16, wherein the second material is a radiopaque material.

18. The medical implant of claim 15, wherein each marker in the plurality of markers comprise a square shape.

19. A method of three-dimensionally printing a medical implant comprising a biocompatible body and an identification assembly, wherein the identification assembly is configured to transmit a data set to a scanning device, the method comprising:
(a) three-dimensionally printing a first portion of the biocompatible body;
(b) three-dimensionally printing a second portion of the biocompatible body and the identification assembly on top of the first portion of the biocompatible body; and
(c) three-dimensionally printing a third portion of the biocompatible body on top of the second portion of the biocompatible body and the identification assembly such that the biocompatible body completely surrounds the identification assembly.

20. The method of claim 19, wherein printing the identification assembly further comprises printing a two-dimensional bar code.
